# EUROPEAN PATENT SPECIFICATION

(11) **EP 0 668 926 B1**
(45) Date of publication and mention of the grant of the patent: **30.06.2004**
(21) Application number: 92925107.2
(22) Date of filing: 12.11.1992
(51) Int. Cl.: C12Q 1/56

(54) **MULTIPLE COAGULATION TEST DEVICE AND METHOD**
TESTVORRICHTUNG UND VERFAHREN FÜR VIELFACHGERINNUNGSTEST
DISPOSITIF ET PROCEDE D'ESSAIS DE COAGULATION MULTIPLES

(43) Date of publication of application: 30.08.1995
(73) Proprietor: GOLDSTEIN, Sheldon, Highland Park, NJ 08904 (US)
(72) Inventor: GOLDSTEIN, Sheldon, Highland Park, NJ 08904 (US)
(74) Representative: Bannerman, David Gardner
(86) International application number: PCT/US1992/009592
(87) International publication number: WO 1994/011527

(56) References cited:
- EP-A- 0 434 377
- US-A- 2 616 796
- US-A- 3 302 452
- US-A- 3 967 934
- US-A- 4 000 972
- US-A- 4 125 327
- US-A- 4 443 408
- US-A- 4 497 774
- US-A- 4 640 896
- US-A- 4 659 550
- US-A- 5 366 869

## Description

### TECHNICAL FIELD

This invention relates to devices for use in quickly determining the causes and treatment for peri-operative or non-surgical hemorrhaging in a patient.

### BACKGROUND

A heart-lung machine is typically used during heart surgery for coronary artery bypass, valvular replacement or proximal aortic reconstruction. Such a machine substitutes for the function of the heart muscle to pump blood throughout the body, and substitutes for lung function by removing carbon dioxide and adding oxygen to the patient's blood.

To use the heart lung machine, full arrest of the coagulation cascade in the blood is required to prevent clot formation on the surfaces of the machine. In the coagulation cascade fibrin formation is initiated with the release from damaged cells of a complex protein known as tissue factor. This initiates the coagulation cascade which ultimately results in fibrin generation. The arrest of the cascade is accomplished typically by administering heparin to the patent. Heparin impedes coagulation by enhancing the effectiveness of anti-thrombin III, a naturally occurring inhibitor of coagulation. It does this by causing a conformational change that exposes additional binding sites on the anti-thrombin III molecule, which increases the ability of antithrombin III to bind with factors XIIa, XIa, IXa and Xa, which in turn accelerates their ability to inhibit the formation of fibrin. After the period of cardiopulmonary bypass is completed, the heparin effect is reversed by administering an antagonist agent, such as protamine.

Determining the proper number of units of heparin to be administered is not easily determined because of two phenomena. First, the amount of heparin that must be injected to achieve a certain plasma heparin concentration varies from patient to patient. Second, a given heparin level does not reflect an exact state of anticoagulation in a particular patient because of a number of factors peculiar to individual patients such as extravascular depots, hemodilution, hypothermia, heparin resistance and anti-thrombin III deficiency.

To determine whether the heparin administered has effectively reduced the ability of the blood to clot, the activated coagulation time (ACT) is measured. The ACT was introduced by Hattersley in 1966 and is a method for the rapid determination of the Lee-White whole blood clotting time. Although initially performed by manual rotation of a test tube and visual inspection for the presence of a clot, the test is typically performed via an automated method known as the HEMOCHRON (International Technidyne, Edison, N.J.). Typically, a sample containing two cc of whole blood is obtained and placed in an ACT tube and the time recorded. The tube is shaken to mix the blood with a diatomaceous powder which promotes coagulation by its high surface area. The tube is then warmed to 37°C. A magnetic rod placed in the tube is observed by a magnetic detector, and when coagulation occurs, the rod is displaced, signalling completion of the test. This test typically takes about 107 ± 13 seconds in a patient with normal coagulation status.

The ACT is currently first measured to provide a baseline ACT and then is measured again after administration of heparin to document whether a safe level of anticoagulation has been attained. The ACT is also measured serially during surgery, usually about every 30 minutes, to be sure that adequate anticoagulation is maintained in the face of metabolism changes and excretion of heparin.

The ACT is also used after surgery is completed. At this time, the heart has been restarted and is pumping blood through the lungs where oxygen is added to the blood and carbon dioxide is removed. The heparin anticoagulation effect is reversed by the administration of an antagonist to heparin, such as protamine. Protamine is polycationic and forms a complex with heparin, thus reversing its effects on Anti-Thrombin III. After administering protamine, the ACT is measured to determine if the protamine has adequately reversed the effects of heparin.

Sometimes, however, the administration of protamine does not return the ACT to the baseline (normal) condition and the patient may experience bleeding. Although many clinicians associate an increased ACT with a prolonged heparin effect, the ACT is limited in that it is a test of essentially the entire coagulation system, and as such, it is affected by other changes in the coagulation cascade. Therefore, an elevated ACT after heparin reversal with protamine does not necessarily indicate that residual heparin is the cause of the elevated ACT. In addition to residual heparin, destruction of serine protease (proteins required for blood to clot, otherwise known as clotting factors) hypofibrinogenemia, fibrinolysis and platelet abnormalities, both qualitative and quantitative, can influence the ACT. Decreased levels of fibronectin, a substance which is involved in platelet adhesion, may also result in increased bleeding.

One device currently used to assist in determining protamine dose is the HEPCON (HemoTec Inc., Englewood, Colorado). The HEPCON device consists of four chambers which contain specific amounts of protamine, thromboplastin and diluent. Air bubbles percolate through a blood sample in each chamber until a photocell detects clot formation in one of the chambers. Based upon the patient's height and weight, the device computes the heparin level. In essence, this device confirms whether or not a patient's bleeding tendency is due to heparin. If protamine administration is followed by obtaining an elevated ACT and a HEPCON test produces a reading of zero, this indicates that no heparin is circulating and it is likely that one or more other etiologies may be responsible for the hemorrhaging.

Bleeding in general surgical or non-surgical patients, including those involved in cardiopulmonary bypass surgery where there is no longer any circulating heparin, could be due to a decreased level of coagulation factors such as factors V, VIII, XIII and fibrinogen, as well as thrombocytopenia, or more commonly, abnormal platelet function, decreased levels of fibronectin, complement activation and fibrinolysis. Decreased levels of serine proteases and platelets could be due to low grade coagulation during bypass with consumption of the factors and platelets, or more likely, damage and destruction sustained when exposed to the surface of the oxygenator.

The anesthesiologist and surgeon are often faced with the situation that a patient is bleeding significantly and it is not due to heparin. A similar situation may occur in patients with massive bleeding due to a medical etiology. Because of the numerous possibilities of which factor or combination of factors is needed to stem the hemorrhaging, combined with the extremely limited amount of time available, the patient is frequently treated with a shotgun therapy, for example, by administration of platelets, fresh frozen plasma, desmopressin acetate (DDAVP) and sometimes epsilo-amino caproic acid (AMICAR) and cryoprecipitate. Since the use of platelets, fresh frozen plasma and cryoprecipitate all carry the risk of disease transmission, a system to rapidly determine if one or two therapies would be sufficient would decrease the risk to the patient of contracting hepatitis, AIDS, and numerous other diseases. Furthermore, in cases where it is determined that DDAVP or Amicar would be therapeutic, the patient would be spared transfusion of blood products altogether. An additional reason to rapidly determine the specific appropriate therapy is that as long as there is a deficiency in blood coagulation, the patient will require transfusion of packed red blood cells (PRBCs). In addition to the risk of disease transmission, transfusion of large amounts of PRBCs dilutes the coagulation factors and platelets in the blood, resulting in a "dilutional coagulopathy", thus possibly worsening the degree of hemorrhaging.

At present, complete, definitive coagulation studies can only be done in the laboratory, which takes too long to be of use in determining a specific therapy against massive hemorrhage associated with an elevated ACT, whether under operating room or non-operating room conditions. There is a need for a method that is rapid enough to allow a doctor to determine and administer a specific therapy under the severe time constraints posed by an episode of rapid massive bleeding whether in the operating room or otherwise.

### SUMMARY OF THE INVENTION

It is an object of the present invention to provide a device which provides specific indications of the particular factors or substances which will positively influence the coagulation effectiveness of whole blood.

It is a further object to provide a device which can be used rapidly yet is sufficiently specific to identify particular factors or substances which can be used to treat intra-operative, post-operative or non-surgical bleeding.

According to the present invention, a clot detection device comprises a plurality of tubes, each of which contains a comparable dosage of a patient's blood. Each tube is contacted with a particular treatment alternative individually. For example, one tube may have an anti-heparin agent such as protamine, or will have fresh frozen plasma or platelets or amicar. The standard ACT test, or other clot detection system will then be carried out, i.e. each tube will include clot detecting means such as a magnetic rod with an associated detector and will be shaken and have their temperature elevated and the effects on coagulation will be assessed. Depending on the most effective treatment for increasing the coagulation effect, a suitable treatment will be determined and applied. Typically, the ACT test takes approximately two minutes and can be done in an operating room without utilizing a separate laboratory facility. Of course, a photocell detector or other detecting means may be used so long as the endpoint is the detection of a fibrin clot in a timed manner.

Utilizing the present invention, a device is provided for immediate operating room or non-operating room use which produces results indicating a proper course of treatment without resort to a shotgun approach which requires an addition of six or more agents to a patient and thus avoids several of the complications inherent in utilizing such an approach. The device thus allows rapid determination of a specific treatment in a hemorrhaging situation without awaiting laboratory test results.

### BRIEF DESCRIPTION OF THE DRAWINGS

The Figure shows one embodiment of the device of the present invention for preforming a multiple coagulation time test.

### DETAILED DESCRIPTION OF THE INVENTION

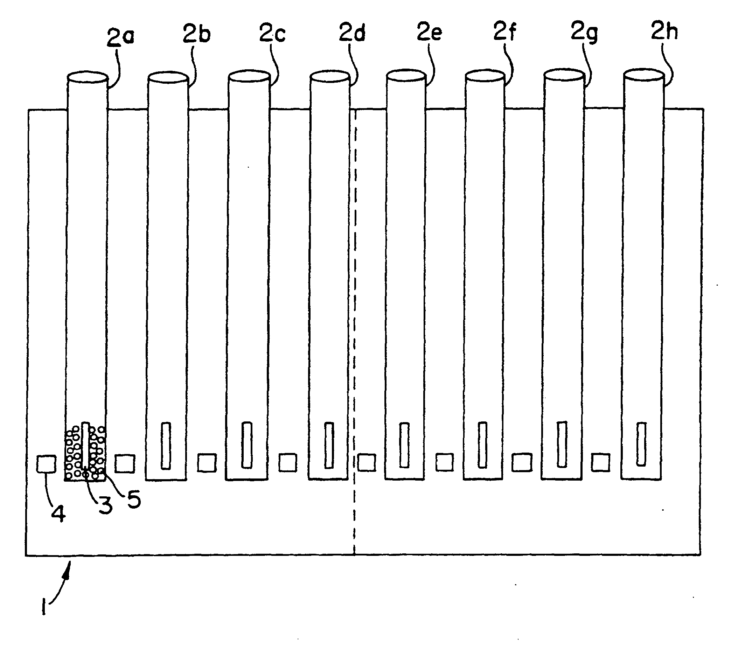

Referring to Fig. 1, a device 1 according to the invention is shown. The device 1 has 8 tubes 2A-H, each tube containing a magnetic rod 3 and having an associated magnetic detector 4. Each tube additionally contains a diatomaceous powder and an individual or combination of treatments for promoting coagulation.

The tubes are preferably sized to accept a 2.5 cc sample of whole blood. To each tube is added a different therapy for coagulation disorders, based upon a standard dose for a 70 kg adult with a blood volume of 5,000 cc. The dose is then divided by 2000 to obtain a dose to be added to each tube.

Tube 2A is a standard and will contain the blood without treatment but after protamine has been administered to the patient. Tube 2B has 25 ucg of protamine. Tube 2C has 0.5 cc of fresh frozen plasma (based upon a dose of 4 units of fresh frozen plasma). Tube 2D has 0.25 cc of platelets, based upon a dose of 10 units of platelets. Tube 2E has 0.1 cc of cryoprecipitate, based upon a dose of 10 units. Tube 2F has 2.5 mg of Amicar, based on a loading dose of 5 grams. Tube 2G has 0.2 cc of desmopressin acetate, based upon a total dose of 0.3mg/kg. Tube 2H contains 0.14 mg/kg of aprotinin. Each tube has 15 mg of diatomaceous earth.

These doses are based on a whole blood sample of 2.5 cc. However, the device may be designed to use the smallest volume of whole blood which still offers accurate test results, for example, a sample size ranging from 0.5 to 2.5 cc could be used. Of course, larger samples may also be used

The initial time is noted when the blood is added to the tubes, which are mixed and heated to 37°C. Preferably, this is done simultaneously. The magnetic detectors then indicate when coagulation occurs and the times will be noted.

In operation, if a patient's ACT after protamine administration remains elevated, the HEPCON is zero, and there is bleeding, the inventive device would be used and a therapy chosen based on the first treatment which brought the ACT closest to its baseline value. If bleeding continues, the treatment which was the next most effective would be administered, etc.

The number of tubes and treatments may be varied considerably. For example, each tube may contain a blood clotting agent such as coagulation factors I (fibrinogen), II (prothrombin), IIa (thrombin), III (thromboplastin), IV, V, VI, VII, VIII, IX, X, XI, XII, recombinant coagulation factors, bovine coagulation factors, coagulation factor VIII:C, Von Willebrand factor, platelets, fibronectin, thrombin, plasminogen, plasminogen activator, plasmin, desmopressin, acetate (DDAVP), epsilo-amino caproic acid (amicar), cryoprecipitate, fresh frozen plasma, protamine, aprotinin and mixtures thereof.

Preferably, 6-10 tubes are incorporated in the device and the individual stop times noted and/or recorded automatically, utilizing suitable instrument control apparatus. Of course, more of less tubes may be used as necessary, as determined in light of experience and most likely or least invasive courses of action. For example, one tube may include both amicar and desmopressin acetate since neither carries the risk of disease transmission.

It is also contemplated that tube pack containing 3-5 tubes each and prefilled with specific treatment agents would be supplied to the user and combined with other packs if needed. Bases on experience, severity of hemorrhaging, etc, a user could select the appropriate pack(s) for a first run in the device and supplement the choice as needed.

Utilizing the inventive device, results are given within about 2 minutes in an operating or emergency room setting, with the results communicated immediately to the attending personnel. Treatment can be given immediately and selectively targeted to give the most effective results. In many cases, risk of disease transmission is reduced as shotgun therapy is avoided.

## Claims

1. A method for determining a treatment for haemorrhaging in a patient to whom heparin has been administered comprising:
a. selecting a plurality of different agents and/or combinations capable of enhancing blood clotting;
b. simultaneously mixing a sample of the patient's blood with each said agent and/or combination of agents;
c. measuring the clotting time for each of said samples;
d. selecting the blood clotting agent or combination of agents that returned the patient's activated coagulation time (ACT) most closely to the baseline figure;
e. wherein each agent or individual combination of agents is kept in a separate sample holding means.

2. The method of claim 1 wherein the blood clotting agents are chosen from coagulation factors, recombinant coagulation factors, bovine coagulation factors, coagulation factor VIII:C. von Willebrand factor, platelets, fibronectin, thrombin, plasminogen, plasminogen activator, plasmin desmopressin acetate (DDAVP), epssilo-amino caproic acid, cryoprecipitate, fresh frozen plasma (FFP), protamine, aprotinin and prostacyclin.

3. The method of claim 2 wherein the coagulation factors are coagulation factors I (fibrinogen), Ia (fibrin), II (prothrombin), IIa (thrombin), III (thromboplastin), IV, V, VI, VII, VIII, IX, X, XI and XII.

4. The method of claim 2 wherein the recombinant factor is recombinant factor VIII.

5. The method of claim 2 wherein the cryoprecipitate is bovine cryoprecipitate, or human cryoprecipitate.

6. The method of claim 2 wherein the fresh frozen plasma is bovine fresh frozen plasma, or human fresh frozen plasma.

7. The method of claim 1 wherein clot formation is detected by incorporating a magnetic rod in each sample holding means and providing a magnetic detector such that when coagulation occurs, the rod is displaced and the detector signals test completion.

8. The method of claim 1 wherein the clotting time is measured by providing a photocell detector and a light source, coagulation interrupting light transmission to signal test completion.

9. A device for determining a treatment in a patient to whom heparin has been administered for haemorrhaging comprising:
A plurality of sample holding means to receive samples of blood from a patient; each holding means having a different agent or combination of agents capable of enhancing clotting of a blood sample introduced therein:
means for simultaneously adding a blood sample to each sample holding means;
means for detecting clotting of the blood samples;
and means for determining the time of clot formation from the addition of the blood samples to the sample holding means;
wherein each agent or individual combination of agents is kept in a separate sample holding mean;
and wherein at least one of said sample holding means is for measuring a baseline clotting indicator time of said patient's blood and contains a sample of blood taken from the patient after administration of a heparin antagonist..

10. The device of claim 9 wherein the sample holding means are tubes.

11. The device of claim 9 wherein the blood clotting agents are chosen from coagulation factors, recombinant coagulation factors, bovine coagulation factors, coagulation factor VIII:C. von Willebrand factor, platelets fibronectin, thrombin, plasminogen, plasminogen activator, plasmin, desmopressin acetate (DDAVP), epsilo-amino caproic acid, cryoprecipitate, fresh frozen plasma (FFP), protamine, aprotinin and prostacyclin.

12. The device of claim I 1 wherein the coagulation factors are coagulation factors I (fibrinogen), Ia (fibrin), II (prothrombin), IIa (thrombin), III (thromboplastin), IV, V, VI, VII, VIII, IX, X, XI, and XII.

13. The device of claim 11 wherein the recombinant factor is recombinant factor VIII.

14. The device of claim 11 wherein the cryoprecipitate is bovine cryoprecipitate, or human cryoprecipitate.

15. The device of claim 11 wherein the fresh frozen plasma is bovine fresh frozen plasma, or human fresh frozen plasma.

16. The device of claim 10 wherein a magnetic rod is located in each tube and associated with a magnetic detector such that when coagulation occurs, the rod is displaced and the detector signals test completion.

17. The device of claim 9 wherein a photocell detector and an associated light source are provided in such a way that coagulation interrupts light transmission from the light source to its associated detector.

18. The device of claim 9 further comprising diatomaceous powder placed in each sample holding means.

19. The device of claim 9 incorporating from 6 to 10 sample holding means.

20. The method of claim 1; wherein said sample holding means are heated to 37°C prior to measuring the clotting time for each said sample.

## Patentansprüche

1. Verfahren zum Bestimmen einer Behandlung von Blutungen in einem Patienten, dem Heparin verabreicht worden ist, umfassend:
a) Auswählen einer Mehrzahl verschiedener Agenzien und/oder Kombinationen, die in der Lage sind, die Blutgerinnung zu verbessern;
b) Gleichzeitiges Vermischen einer Probe des Blutes des Patienten mit jedem dieser Agenzien und/oder Kombination von Agenzien;
c) Messen der Gerinnungszeit für jede der Proben;
d) Auswählen des Blutgerinnungsagens oder der Kombination von Agenzien, mit denen die aktivierte Koagulationszeit (ACT) des Patienten am nähesten zur Grundlinie zurückkehrte;
e) wobei jedes Agens oder jede individuelle Kombination von Agenzien in einer separaten Probehaltevorrichtung aufbewahrt wird.

2. Verfahren nach Anspruch 1, wobei die Blutgerinnungsagenzien ausgewählt sind aus Koagulationsfaktoren, rekombinanten Koagulationsfaktoren, bovinen Koagulationsfaktoren, Koagulationsfaktor VIII: C. von Willebrand-Faktor, Plättchen, Fibronektin, Thrombin, Plasminogen, Plasminogenaktivator, Plasmin, Desmopressinacetat (DDAVP), Epsilon-Aminokapronsäure, Kryopräzipitat, frisches gefrorenes Plasma (FFP), Protamin, Aprotinin und Prostacyclin.

3. Verfahren nach Anspruch 2, wobei die Koagulationsfaktoren Koagulationsfaktoren I (Fibrinogen), Ia (Fibrin), II (Prothrombin), IIa (Thrombin), III (Thromboplastin), IV, V, VI, VII, VIII, IX, X, XI und XII sind.

4. Verfahren nach Anspruch 2, wobei der rekombinante Faktor rekombinanter Faktor XIII ist.

5. Verfahren nach Anspruch 2, wobei das Kryopräzipitat bovines Kryopräzipitat oder humanes Kryopräzipitat ist.

6. Verfahren nach Anspruch 2, wobei das frische gefrorene Plasma bovines frisches gefrorenes Plasma oder humanes frisches gefrorenes Plasma ist.

7. Verfahren nach Anspruch 1, wobei die Gerinnungsbildung durch Einbringen eines Magnetstabes in jede Probehaltevorrichtung und Bereitstellen eines Magnetdetektors derart nachgewiesen wird, dass bei Eintreten der Koagulation der Stab verdrängt wird und der Detektor Beendigung des Tests signalisiert.

8. Verfahren nach Anspruch 1, wobei die Gerinnungszeit dadurch gemessen wird, dass ein Fotozelldetektor und eine Lichtquelle bereitgestellt werden, wobei die Koagulation die Lichtübertragung unterbricht und so das Testende signalisiert.

9. Vorrichtung zum Bestimmen einer Behandlung in einem Patienten, dem Heparin verabreicht worden ist, gegen Blutungen, umfassend:
Eine Mehrzahl von Probehaltevorrichtungen zur Aufnahme von Blutproben eines Patienten; wobei jede Haltevorrichtung ein verschiedenes Agens oder Kombination von Agenzien aufweist, die in der Lage sind, die Gerinnung einer darin eingeführten Blutprobe zu verbessern;
Mittel für eine gleichzeitige Zugabe einer Blutprobe zu einer jeden Probehaltevorrichtung;
Mittel zum Nachweis der Gerinnung der Blutproben;
und Mittel zum Bestimmen der Zeit der Gerinnungsbildung von der Zugabe der Blutproben zu den Probehaltevorrichtungen;
wobei jedes Mittel oder individuelle Kombination von Mitteln in einer separaten Probehaltevorrichtung aufbewahrt wird;
und wobei mindestens eine der Probehaltevorrichtungen zum Messen einer Grundliniengerinnungsindikatorzeit des Blutes des Patienten vorgesehen ist und eine Blutprobe enthält, die dem Patienten nach Verabreichung eines Heparin-Antagonisten entnommen wurde.

10. Vorrichtung nach Anspruch 9, wobei die Probehaltevorrichtungen Röhrchen sind.

11. Vorrichtung nach Anspruch 9, wobei die Blutgerinnungsagenzien ausgewählt sind aus Koagulätionsfaktoren, rekombinanten Koagulationsfaktoren, bovinen Koagulationsfaktoren, Koagulationsfaktor VIII: C. von Willebrand-Faktor, Plättchen, Fibronektin, Thrombin, Plasminogen, Plasminogenaktivator, Plasmin, Desmopressinacetat (DDAVP), Epsilon-Aminokapronsäure, Kryopräzipitat, frisches gefrorenes Plasma (FFP), Protamin, Aprotinin und Prostacyclin.

12. Vorrichtung nach Anspruch 11, wobei die Koagulationsfaktoren Koagulationsfaktoren I (Fibrinogen), la (Fibrin), 11 (Prothrombin), IIa (Thrombin), III (Thromboplastin), IV, V, VI, VII, VIII, IX, X, XI und XII sind.

13. Vorrichtung nach Anspruch 11, wobei der rekombinante Faktor rekombinanter Faktor XIII ist.

14. Vorrichtung nach Anspruch 11, wobei das Kryopräzipitat bovines Kryopräzipitat oder humanes Kryopräzipitat ist.

15. Vorrichtung nach Anspruch 11, wobei das frische gefrorene Plasma bovines frisches gefrorenes Plasma oder humanes frisches gefrorenes Plasma ist.

16. Vorrichtung nach Anspruch 10, wobei ein Magnetstab in jedem Röhrchen platziert wird und mit einem Magnetdetektor derart verbunden wird, dass bei Eintreten der Koagulation das Stäbchen verdrängt wird und der Detektor die Beendigung des Tests signalisiert.

17. Vorrichtung nach Anspruch 9, wobei ein Fotozelldetektor und eine verbundene Lichtquelle auf solche Weise bereit gestellt werden, dass Koagulation die Lichttransmission von der Lichtquelle zu dem mit ihr verbundenen Detektor unterbricht.

18. Vorrichtung nach Anspruch 9, darüber hinaus umfassend Diatomeenpulver, das in jede der Probehaltevorrichtungen gegeben wird.

19. Vorrichtung nach Anspruch 9, enthaltend 6-10 Probehaltevorrichtungen.

20. Verfahren nach Anspruch 1, wobei die Probehaltevorrichtungen vor Messung der Gerinnungszeit für jede der Proben auf 37°C erwärmt werden.

## Revendications

1. Procédé de détermination d'un traitement de l'hémorragie chez un patient auquel de l'héparine a été administrée, comprenant :
a) la sélection de plusieurs agents différents et/ou combinaisons différentes destinés à accroître le figement du sang,
b) le mélange simultané d'un échantillon du sang du patient à chacun des agents et/ou combinaison d'agents,
c) la mesure du temps de figement pour chacun des échantillons, et
d) la sélection de l'agent de figement du sang ou d'une combinaison d'agents qui approche le temps de coagulation activée du patient (ACT) le plus près de la valeur de référence,
e) chaque agent ou combinaison individuelle d'agents étant maintenu dans un dispositif séparé de maintien d'échantillons.

2. Procédé selon la revendication 1, dans lequel les agents de figement du sang sont choisis parmi les facteurs de coagulation, les facteurs recombinant de coagulation, les facteurs de coagulation bovins, le facteur de coagulation VIII:C, le facteur de von Willebrand, les plaquettes, la fibronectine, la thrombine, le plasminogène, l'activateur de plasminogène, l'acétate de plasmine-desmoprécine (DDAVP), l'acide ε-aminocaproïque, un cryoprécipité, du plasma frais congelé (FFP), de la protamine, de l'aprotinine et de la prostacycline.

3. Procédé selon la revendication 2, dans lequel les facteurs de coagulation sont les facteurs de coagulation I (fibrinogène), Ia (fibrine), II (prothrombine), IIa (thrombine), III (thromboplastine), IV, V, VI, VII, VIII, IX, X, XI et XII.

4. Procédé selon la revendication 2, **caractérisé en ce que** le facteur recombinant est le facteur recombinant VIII.

5. Procédé selon la revendication 2, dans lequel le cryoprécipité est un cryoprécipité bovin ou humain.

6. Procédé selon la revendication 2, dans lequel le plasma frais congelé est du plasma frais congelé bovin ou humain.

7. Procédé selon la revendication 1, dans lequel la formation d'un caillot est détectée par incorporation d'un barreau magnétique dans chaque dispositif de maintien d'échantillons et par disposition d'un détecteur magnétique tel que, lorsque la coagulation se produit, le barreau est déplacé et le détecteur signale la fin du test.

8. Procédé selon la revendication 1, dans lequel le temps de figement est mesuré par disposition d'un détecteur à cellule photoélectrique et d'une source de lumière, la coagulation interrompant la transmission de lumière pour signaler la fin du test.

9. Dispositif de détermination d'un traitement dans un patient auquel de l'héparine a été administrée pour lutter contre l'hémorragie, comprenant :
plusieurs dispositifs de maintien d'échantillons destinés à recevoir des échantillons de sang d'un patient, chaque dispositif de maintien ayant un agent différent ou une combinaison différente d'agents capable d'augmenter le figement d'un échantillon sanguin introduit dans le dispositif,
un dispositif d'addition simultané d'un échantillon de sang à chaque dispositif de maintien d'échantillons,
un dispositif de détection du figement des échantillons sanguins, et
un dispositif de détermination du temps de formation d'un caillot depuis l'addition des échantillons de sang au dispositif de maintien d'échantillons,
dans lequel chaque agent ou chaque combinaison individuelle d'agents est maintenu dans un dispositif séparé de maintien d'échantillons, et
dans lequel au moins un des dispositifs de maintien d'échantillons est destiné à la mesure d'un temps indicateur de figement de référence du sang du patient et contient un échantillon de sang prélevé sur le patient après l'administration d'un antagoniste d'héparine.

10. Dispositif selon la revendication 9, dans lequel les dispositifs de maintien d'échantillons sont des tubes.

11. Dispositif selon la revendication 9, dans lequel les agents de figement du sang sont choisis parmi les facteurs de coagulation, les facteurs recombinant de coagulation, les facteurs de coagulation bovins, le facteur de coagulation VIII:C, le facteur de von Willebrand, les plaquettes, la fibronectine, la thrombine, le plasminogène, l'activateur de plasminogène, l'acétate de plasmine-desmoprécine (DDAVP), l'acide ε-aminocaproïque, un cryoprécipité, du plasma frais congelé (FFP), de la protamine, de l'aprotinine et de la prostacycline.

12. Dispositif selon la revendication 11, dans lequel les facteurs de coagulation sont les facteurs de coagulation I (fibrinogène), Ia (fibrine), II (prothrombine), IIa (thrombine), III (thromboplastine), IV, V, VI, VII, VIII, IX, X, XI et XII.

13. Dispositif selon la revendication 11, dans lequel le facteur recombinant est le facteur recombinant VIII.

14. Dispositif selon la revendication 11, dans lequel le cryoprécipité est un cryoprécipité bovin ou humain.

15. Dispositif selon la revendication 11, dans lequel le plasma frais congelé est du plasma frais congelé bovin ou humain.

16. Dispositif selon la revendication 10, dans lequel un barreau magnétique est placé dans chaque tube et est associé à un détecteur magnétique de manière que, lorsque la coagulation se produit, le barreau est déplacé et le détecteur indique la fin du test.

17. Dispositif selon la revendication 9, dans lequel un détecteur à cellules photoélectriques et une source de lumière associée sont disposés de manière que la coagulation interrompe la transmission de lumière de la source de lumière au détecteur associé.

18. Dispositif selon la revendication 9, contenant en outre une poudre de diatomées placée dans chaque dispositif de maintien d'échantillons.

19. Dispositif selon la revendication 9, contenant six à dix dispositifs de maintien d'échantillons.

20. Dispositif selon la revendication 1, dans lequel les dispositifs de maintien d'échantillons sont chauffés à 37 °C avant la mesure du temps de figement pour chaque échantillon.
